(19) Europäisches Patentamt
European Patent Office
Office européen des brevets

(11) Veröffentlichungsnummer: **0 084 118**
**B1**

(12) # EUROPÄISCHE PATENTSCHRIFT

(45) Veröffentlichungstag der Patentschrift: **09.04.86**

(51) Int. Cl.⁴: **C 07 D 213/20**, C 07 D 213/61, C 07 D 213/79

(21) Anmeldenummer: **82111419.6**

(22) Anmeldetag: **09.12.82**

(54) **Verfahren zur Herstellung von 2-Halogenpyridinderivaten.**

(30) Priorität: **14.01.82 CH 194/82**

(43) Veröffentlichungstag der Anmeldung: **27.07.83 Patentblatt 83/30**

(45) Bekanntmachung des Hinweises auf die Patenterteilung: **09.04.86 Patentblatt 86/15**

(84) Benannte Vertragsstaaten: **AT BE CH DE FR GB IT LI NL SE**

(56) Entgegenhaltungen: **Keine**

(73) Patentinhaber: **LONZA AG, Gampel/Wallis (CH)**

(72) Erfinder: **Quarroz, Daniel Dr., Balfrinstrasse 21, Visp (Kanton Wallis) (CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

**EP 0 084 118 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 2-Halogenpyridinderivaten der allgemeinen Formel I

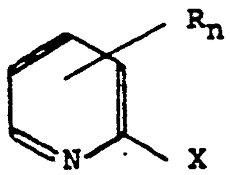

(I)

in welcher X ein Halogenatom, R eine -COOH-Gruppe oder einen niedrigen Alkylrest und n eine Zahl zwischen 0 und 4 bedeutet. 2-Halogenpyridinverbindungen sind zahlreichen Folgereaktionen zugänglich, da das Halogen in 2-Stellung relativ leicht austauschbar ist. So zum Beispiel dient die 2-Chlor-5-pyridin-carbonsäure (6-Chlornicotinsäure) als Ausgangsmaterial zur Herstellung von 2-Amino-5-pyridincarbonsäureester und -amid (CA 90, 174689 c, CA 88, 141704 m). Ein Folgeprodukt des 2-Chlorpyridins ist das Pyridin-2-thiol-1-oxid (Omadine), ein Mittel gegen Kopfhautschuppen. Ferner ist das 2-Chlor-pyridin ein wichtiges Zwischenprodukt für zahlreiche Arzneimittel, z.B. für die Antihistaminika vom Phenivamin-Typ. Es ist bekannt, 2-Chlorpyridinderivate, z.B. 2-Chlor-5-pyridincarbonsäure (6-Chlornicotinsäure), durch Chlorierung von 2-Hydroxy-5-pyridincarbonsäure (6-Hydroxynicotinsäure) mit $PCl_5/POCl_3$ herzustellen, wobei die Hydroxynicotinsäure z.B. aus Äpfelsäure hergestellt wird. Dieses Verfahren ist kompliziert und bringt durch die Verwendung von Chlorphosphorverbindungen erhebliche Abwasserprobleme.

2-Chlorpyridin wird nach bekannten Verfahren durch Gasphasenchlorierung von Pyridin in Gegenwart von Wasser (US 3920657) oder Tetrachlorkohlenstoff (US 3153044) bei Temperaturen um 350°C hergestellt. Die Pyridinumsätze liegen nur bei 34-50%. Nichtumgesetztes Pyridin fällt als Hydrochlorid an und muss vor der Wiederverwendung aufgearbeitet werden. Daneben entstehen noch andere Chlorierungsprodukte, die vom gewünschten 2-Chlorpyridin abgetrennt werden müssen.

Ziel der vorliegenden Erfindung war es, auf einfache Weise 2-Halogenpyridinderivate, insbesondere 2-Chlorpyridin und 2-Chlor-5-pyridincarbonsäure, herzustellen.

Erfindungsgemäss wird dies dadurch erreicht, dass man die entsprechenden 2-Pyridincarbonsäure-N-oxid-derivate der allgemeinen Formel II

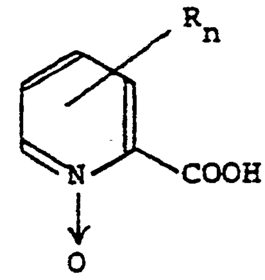

(II)

in welcher R = COOH oder einen niedrigen Alkylrest (d.h. mit 1 - 6, insbesondere 1 - 4 Kohlenstoffatomen) und n eine Zahl zwischen 0 - 4 bedeuten,

in Gegenwart von organischen Säureanhydriden, tertiären Alkylaminen und einer halogenliefernden Verbindung der allgemeinen Formel III

$CH_nX_m$ (III)

wobei n = 1 oder 2 und m = 2 oder 3,

X = Halogen und m + n = 4 ist,

oder der allgemeinen Formel IV

$C_2H_pX_m$ (IV)

wobei p = 3 oder 4 und m = 2 oder 3,

X = Halogen und p + m = 6 ist,

im Ueberschuss umsetzt, das Umsetzungsgemisch auf einen pH-Wert von 12 - 13 einstellt und aus diesem Gemisch das gewünschte Produkt isoliert.

Im Falle, dass R = Alkylrest(e) bedeutet, wird die Isolierung zweckmässig durch Extraktion bewerkstelligt. Im Falle, dass R = COOH-Rest(e) bedeutet, erfolgt die Isolierung zweckmässig durch Einstellen des Umsetzungsgemisches auf einen pH-Wert von 1,5 - 2, wobei die 2-Halogenpyridincarbonsäure ausfällt.

Als Ausgangsprodukte kommen die N-oxide der 2-Pyridincarbonsäure selbst oder die N-Oxide der Derivate in Betracht, die in 3-, 4-, 5- oder 6-Stellung weitere Substituenten (= R) aufweisen. Solche N-oxide sind beispielsweise die der Isocinchomeronsäure, Chinolinsäure, Lutidinsäure, Dipicolinsäure, α-Carbocinchomeronsäure, α-Carbodinicotinsäure, α'-Carbisocinchomeronsäure, Berberonsäure, 6-Methylpicolinsäure, 6-Ethylpicolin-säure, 4-Methylpicolinsäure u.a.. Vorzugsweise wird das N-oxid der Isocinchomeronsäure zur Herstellung von 6-Chlor-nicotinsäure und das N-oxid der Picolinsäure zur Herstellung von 2-Chlorpyridin verwendet. Als organische Säureanhydride werden gemischte Anhydride oder reine Anhydride aliphatischer Carbonsäuren mit 1-4 C-Atomen eingesetzt. Solche sind beispielsweise Ameisensäure-essigsäureanhydrid, Essigsäurepropionsäureanhydrid, Essigsäurebuttersäureanhydrid oder als reine Anhydride solche der Essigsäure, Propionsäure, Buttersäure. Vorzugsweise wird Essigsäureanhydrid verwendet.

Die Menge der Säureanhydride ist

vorzugsweise so zu wählen, dass ein leichter Ueberschuss von vorteilhaft 1,1 - 3 Äquivalenten pro Äquivalent N-Oxid im Reaktionsgemisch vorhanden ist.

Als tertiäre Alkylamine kommen solche auf der Basis von Trialkylaminen mit 1-4 C-Atomen in jeder Alkylgruppe zur Anwendung. Solche sind Trimethyl-, Triethyl-, Tripropyl-, Tributylamin. Vorzugsweise wird Triethylamin in Mengen von zweckmässig 2 - 3 Äquivalenten pro Äquivalent N-oxid eingesetzt.

Als halogenliefernde Verbindungen kommen die Halogenalkane in Betracht, deren H-Atome noch nicht vollständig durch Halogen ausgetauscht sind. Solche sind $CHCl_3$, $CH_2Cl_2$, $CHCl_2$-$CH_2Cl$, $CH_3$-$CHCl_2$, $CH_2Br_2$, $CH_2J_2$. Vorzugsweise kommen $CH_2Cl_2$, $CH_2Br_2$ und $CH_2J_2$ zum Einsatz.

Es ist wichtig, diese halogenliefernden Verbindungen im Ueberschuss anzuwenden. Vorzugsweise wird eine Menge von 8 - 60 Äquivalenten pro Äquivalent N-oxid verwendet.

Nach dem erfindungsgemässen Verfahren wird ein Gemisch von 2-Halogen- und 2-Hydroxyverbindungen erhalten. Je grösser der Ueberschuss an halogenlieferndes Verbindung, um so grösser ist die Bildung der gewünschten 2-Halogenverbindung.

Die Temperatur hat praktisch keinen Einfluss auf die Ausbeute; vorzugsweise wird die Reaktion aber bei Temperaturen von 0 - 65°C durchgeführt. Bei Anwendung von höheren Temperaturen (z.B. 60 - 65°C, $CH_2Cl_2$) wird vorteilhaft unter Druck gearbeitet.

Das Verfahren der Erfindung kann ohne oder in Gegenwart eines fremden Lösungsmittels durchgeführt werden. Vorteilhaft wird kein fremdes Lösungsmittel angewendet, d.h. die Ueberschüsse an Säureanhydrid, Alkylamin und insbesondere an halogenliefernder Verbindung wirken selbst als Lösungsmittel.

Das Verfahren der Erfindun wird zweckmässig so durchgeführt, dass man entweder Säureanhydrid, Alkylamin und halogenliefernde Verbindung vorlegt und das N-oxid zudosiert der N-oxid, Alkylamin und halogenliefernde Verbindung vorlegt und das Säureanhydrid zudosiert. Die Aufarbeitung dieses Reaktionsgemisches kann auf zweierlei Arten geschehen. Nach der einen Methode destilliert man nach dem Zudosieren des N-oxids oder des Säureanhydrids das überschüssige Säureanhydrid, die halogenliefernde Verbindung und das Alkylamin ab und stellt den pH-Wert der Lösung auf einen Wert von 12 - 13 ein. Dies geschieht vorzugsweise durch Zugabe eines Alkalihydroxids, vorzugsweise NaOH.

Das dabei in Freiheit gesetzte Alkylamin wird abdestilliert und die wässrige Lösung zur Gewinnung der alkylierten 2-Halogen-pyridine extrahiert. Zur Isolierung der 2-Halogenpyridincarbonsäure wird die wässrige Lösung, vorzugsweise mit Salzsäure, auf den isoelektrischen Punkt gebracht. Dabei fällt ein Gemisch von 2-Halogen- und 2-Hydroxypyridincarbonsäureverbin-dung aus, das anschliessend durch fraktionierte Kristallisation getrennt werden kann.

Zur Herstellung von 2-Halopyridincarbonsäure nach einer anderen Methode wird nach Zudosieren des N-oxids oder des Säureanhydrids das Reaktionsgemisch ohne vorangehende Destillation auf einen pH-Wert von 12 - 13 gebracht, die organische Phase abgezogen und in einen nächsten Ansatz rezirkuliert. Die wässrige Lösung wird angesäuert und wie oben beschrieben weiter behandelt.

Die Ausgangsverbindungen können auf bekannte Weise hergestellt werden. So zum Beispiel wird zur Herstellung des N-oxids der Isocinchomeronsäure von 2-Methyl-5-ethylpyridin ausgegangen, das in die Isocinchomeronsäure durch Oxidation übergeführt und letzlich durch Behandeln mit $H_2O_2$ in das N-oxid umgesetzt wird. Die Ausgangsverbindungen sind daher leicht zugänglich.

**Beispiel 1**

Herstellung von 6-Chlornicotinsäure (6-ClNS) und 6-Hydroxy-nicotinsäure (6-HNS) aus Isocinchomeronsäure-N-oxid.

15,3 g Essigsäureanhydrid (0,15 Mol), 30 g Triethylamin (0,3 Mol) und 200 g $CH_2Cl_2$ (2,35 Mol) wurden in einem Kolben vorgelegt. 1ß,3 g Isocinchomeronsäure-N-oxid (0,1 Mol) wurden bei 40°C portionenweise so zudosiert, dass sich $CO_2$ lebhaft entwickelte. Nach beendeter Zugabe liess man noch ca. 1 Std. bei 40°C nachreagieren, bis kein $CO_2$ mehr entwich. Die resultierende leicht braune Lösung wurde am Rotavapor eingeengt und der dickflüssige Rückstand durch Zusatz von 10%-iger NaOH (End-pH-Wert ungef. 13) bei 80°C 1 Std. behandelt.

Zur Entfernung des Triethylamins wurde das flüssige Reaktionsgemisch abdestilliert und anschliessend mit konzentrierter HCl angesäuert (pH-Wert 1,5). Der hierbei angefallene Niederschlag wurde abgenutscht, mit $H_2O$ gewaschen und bei 45°C, 20 Torr getrocknet.

Ausbeute: 13,1 g Produktegemisch (Gehalt an Nicotinylderivaten 97,2%, Molverhältnis ClNS/HNS = 55/45), entsprechend einer Ausbeute von 47% an 2-Chlor-5-pyridincarbonsäure (6-Chlornicotin-säure) und 38,4% an 2-Hydroxy-5-pyridincarbonsäure (6-Hydroxynicotinsäure), bezogen auf eingesetztes Isocinchomeronsäure-Noxid.

**Beispiel 2**

Es wurde wie in Beispiel 1 vorgegangen, aber mit 500 g $CH_2Cl_2$ und 0,3 Mol Triethylamin.

Ausbeute: 13,9 g Produktgemisch (Gehalt an Nicotinylderivaten 98,5%, Molverhältnis ClNS/HNS = 64/36), entsprechend einer Ausbeute von 58,1% 6-Chlornicotinsäure und

32,7% 6-Hydroxy-nicotinsäure, bezogen auf Isocinchomeronsäure-N-oxid.

**Beispiel 3**

Es wurde wie in Beispiel 1 vorgegangen, aber die Reaktion bei 0 - 5°C geführt.
Ansatz: 25,5 g $Ac_20$, 20 g $Et_3N$, 350 g $CH_2Cl_2$ und 18,3 g Isocinchomeronsäure-N-oxid.
Ausbeute: 53,8% 6-Chlornicotinsäure (2-Chlor-5-pyridincarbon-säure)
39,0% 6-Hydroxynicotinsäure (2-Hydroxy-5-pyridincarbonsäure)

**Beispiel 4**

Eine Aufschlämmung von 50 g $CH_2Cl_2$, 10 g $Et_3N$ und 9,1 g Isocinchomeronsäure-N-oxid wurden in einem Autoklav vorgelegt. Unter 4,5 atü und bei 65°C pumpte man ein Gemisch von 10,2 g $Ac_2O$, 5 g $Et_3N$ und 50 g $CH_2Cl_2$ zu. Am Ende der Zugabe wurde das Reaktionsgemisch wie in Beispiel 1 aufgearbeitet.
Ausbeute: 40,8% 2-Chlor-5-pyridincarbonsäure (6-Chlornicotin-säure)
44,2% 2-Hydroxy-5-pyridincarbonsäure (6-Hydroxynicotinsäure)

**Beispiel 5**

36,6 g $Ac_20$, 60 g $Et_3N$ und 600 g $CH_2Cl_2$ wurden in einem Kolben vorgelegt und bei 40°C aufgeheizt. 36,6 g Isocinchomeronsäure-N-oxid wurden portionenweise zudosiert. Nach 1,5 Std. Nachreaktion wurde unter starkem Rühren NaOH 10% zugegeben, bis der End-pH-Wert 13 erreichte.

Die Phasen wurden abgetrennt. Die $CH_2Cl_2$-Lösung wurde mit festem NaOH getrocknet und für den nächsten Ansatz aufbewahrt. Die wässrige Lösung wurde mit HCl konz. angesäuert (End-pH-Wert ungef. 2).

Das hierbei angefallene Produkt wurde abgenutscht, gewaschen und getrocknet.
Ausbeute: 54,2% 2 Chlor-5-pyridincarbonsäure (6-Chlornicotin-säure)
37,7% 2-Hydroxy-5-pyridincarbonsäure (6-Hydroxynicotinsäure)

Der getrockneten $CH_2Cl_2$-Phase (enthielt $Et_3N$) gab man 30,6 g $Ac_2O$ und 6 g $Et_3N$ zu und man ergänzte mit $CH_2Cl_2$ bis zum Volumen des ersten Ansatzes. Dann wurden 36,6 g Isocinchomeronsäure-N-oxid langsam bei 40°C zugegeben. Wie oben wurden die Nicotinylderivate isoliert.
Ausbeute: gleich wie oben, bezogen auf neu eingesetztes Isocinchomeronsäure-N-oxid.

**Beispiele 6 - 12**

| | Ausgangsmaterial | Halog. Verb. | Säureanhydrid | Alkylamin | Temp. | Ausbeute |
|---|---|---|---|---|---|---|
| 6 | Chinolinsäure-N-oxid 0,1 mol | $CHCl_3$ 500 g | Essigsäure-buttersäure-anhydrid | Tri-ethyl-amin | 40°C | 47% 2-Chlo nicotinsäu 7% 2-Hydro nicotinsäu |
| 7 | Lutidinsäure-N-oxid 0,2 mol | $CH_2Cl_2$ 600 g | Essigsäure-anhydrid | Tri-butyl-amin | 40°C | 48%2-Chlor-isonicotin-säure, 26% 2-Hydroxy-isonicotin: |
| 8 | Dipicolinsäure-N-oxid 0,2 mol | $CH_2Cl-CHCl_2$ 600 g | Essigsäure-anhydrid | Tri-ethyl amin | 40°C | 13% 2-Chlor pyridincarb 57% 2-Hydro pyridincarb |
| 9 | 6-Methylpico-linsäure-N-oxid 0,1 mol | $CH_2Cl_2$ 200 g | Essigsäure-anhydrid | Tri-ethyl-amin | 40°C | 24% 2-Chlor 6-picolin |
| 10 | Picolinsäure N-oxid 0,1 mol | $CH_2Cl_2$ 200 g | Essigsäure-anhydrid | Tri-ethyl-amin | 40°C | 50% 2-Chlor pyridin |
| 11 | Isoinchomeron-säure-N-oxid 0,1 mol | $CH_2Br_2$ 400 g | Essigsäure-anhydrid | Tri-ethyl-amin | 40°C | 56% 2-Brom-5-pyridincarbon-säure, 26,6% droxy-5-pyri carbonsäure |
| 12 | Isoinchomeron-säure-N-oxid 0,1 mol | $CH_2I_2$ 500 g | Essigsäure-anhydrid | Tri-ethyl-amin | 40°C | 35% 2-Jod-5-pyridincarbo säure, 15% 2 Hydroxy-5-py dincarbonsäu |

**Patentansprüche**

1. Verfahren zur Herstellung von 2-Halogenpyridinderivaten der allgemeinen Formel I

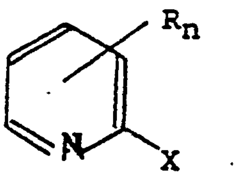

(I)

in welcher X ein Halogenatom, R eine COOH-Gruppe oder einen niedrigen. Alkylrest und n eine Zahl zwischen 0 und 4 bedeuten, dadurch gekennzeichnet, dass man die entsprechenden 2-Pyridincarbonsäure-N-oxid-derivate der allgemeinen Formel II

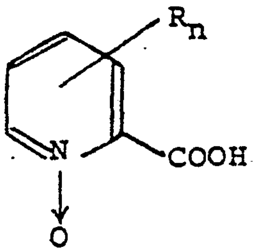

(II)

in Gegenwart von organischen Säureanhydriden aliphatischer Carbonsäuren mit 1 bis 4 C-Atomen, tertiären Alkylaminen auf der Basis von Trialkylaminen mit 1 bis 4 C-Atomen in jeder Alkylgruppe und einer halogenliefernden Verbindung der allgemeinen Formel III

$CH_nX_m$ III

wobei n = 1 oder 2 und m = 2 oder 3,

X=Halogen und n + m = 4 ist;

oder der allgemeinen Formel IV

$C_2H_pX_m$II

wobei p = 3 oder 4 und m = 2 oder 3,

X=Halogen und p + m = 6 ist,

im Ueberschuss umsetzt, das Imsetzungsgemisch auf einen pH-Wert von 12 - 3 einstellt und aus diesem Gemisch das ewünschte Produkt isoliert.

2. Verfahren nach Patentanspruch 1, dadurch ekennzeichnet, dass man als organisches äureanhydrid Essigsäureanhydrid verwendet.

3. Verfahren nach Patentansprüchen 1 und 2, adurch gekennzeichnet, dass man als tertiäres lkylamin Triethylamin verwendet.

4. Verfahren nach Patentansprüchen 1 - 3, adurch gekennzeichnet, dass man als alogenliefernde Verbindung $CH_2X_2$ mit X gleich hlor, Jod oder Brom verwendet.

5. Verfahren nach Patentansprüchen 1 - 4, dadurch gekennzeichnet, dass man die halogenliefernde Verbindung in einem Ueberschuss von 8 - 60 Äquivalenten, bezogen auf 1 Äquivalent N-oxid, verwendet.

6. Verfahren nach Paten nsprüchen 1 - 5, dadurch gekennzeichnet, dass man das Säureanhydrid in einem Ueberschuss von 1,2 - 3 Äquivalenten, bezogen auf 1 Äquivalent N-oxid, verwendet.

7. Verfahren nach Patentansprüchen 1 - 6, dadurch gekennzeichnet, dass man das Säureanhydrid zusammen mit dem tertiären Alkylamin und der halogenliefernden Verbindung vorlegt und das N-Oxid zudosiert.

8. Verfahren nach Patentansprüchen 1 - 6, dadurch gekennzeichnet, dass man das N-oxid zusammen mit dem tertiären Alkylamin und der halogenliefernden Verbindung vorlegt und das Säureanhydrid zudosiert.

9. Verfahren nach Patentansprüchen 1 - 8, dadurch gekennzeichnet dass man im Falle dass R = Alkylrest(e) bedeutet, die 2-Halogenpyridine durch Extraktion aus dem Umsetzungsgemisch isoliert.

10. Verfahren nach Patentansprüchen 1 - 8, dadurch gekennzeichnet, dass man im Falle, dass R = COOH-Gruppe(n) bedeutet, die 2-Halogenpyridincarbonsäure durch Einstellen des Umsetzungsgemisches auf einen pH-Wert von 1,5 bis 2 ausfällt.

**Claims**

1.- Process for preparing 2-halopyridine derivates of the general formula I

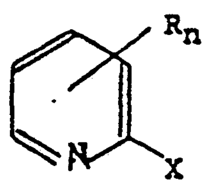

(I)

wherein X is a halogen atom, R is a COOH-group or a lower alkyl radical, and n is an integer between 0 and 4, characterized in that the respective 2-pyridine carboxylic acid-Noxide derivatives of the general formula II

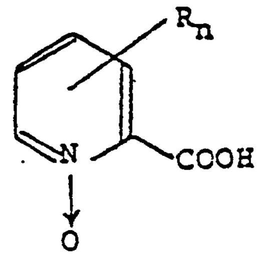

(II)

are reacted in the presence of organic acid anhydrides of aliphatic carboxylic acids having I to 4 carbon atoms, tertiary alkyl amines on the basis of trialkyl amines having I to 4 carbon atoms in each alkyl group and an excess of a halogen providing compound of the general formula III

$CH_nX_m$m (III)

wherein:

n being I or 2, and m being 2 or 3, X being halogen and n + m being 4;

or of the general formula IV

$C_2H_pX_m$ (IV)

wherein:

p being 3 or 4, m being 2 or 3, x being halogen and p + m being 6, the reaction mixture being adjusted to a pH of 12 to 13, and the desired products are isolated from said mixture.

2.- Process according to claim I, characterized in that, as organic acid anhydride,acetic acid anhydride is used.

3.- Process according to claims I and 2, characterized in that as tertiary alkyl amine,triethyl amine is used.

4.- Process according to claims I to 3, characterized in that as halogen providing compound $CH_2X_2$ is used, X being chlorine, iodine or bromine.

5.- Process according to claims I to 4, characterized in that the halogen providing compound is used in an excess of 8 to 60 equivalents, based on I equivalent of the N-oxide.

6.- Process according to claims I to 5, characterized in that the acid anhydride is used in an excess of 1.2 to 3 equivalents, based on I equivalent of the N-oxide.

7.- Process according to claims I to 6, characterized in that the N-oxide is added to the acid anhydride which is present together with the tertiary amine and the halogen providing compound.

8.- Process according to claims I to 6, characterized in that the acid anhydride is added to the N-oxide which is present together with the tertiary amine and the halogen providing compound.

9.- Process according to claims I to 8, characterized in that the 2-halopyridines are isolated from the reaction mixture by extraction in the case of R being alkyl radical(s).

10.- Process according to claims 1 to 8, characterized in that the 2-halopyridine carboxylic acid is precipitated by adjusting the reaction mixture to a pH of 1.5 to 2 in the case of R being one or several COOH group(s).

**Revendications**

1. Procédé pour la préparation de dérivés de 2-halogénopyridines de formule générale I

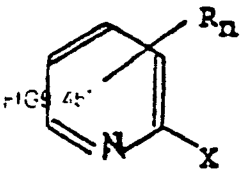

(I)

dans laquelle X représente un atome d'halogène, R un groupe COOH, ou un radical alcoyle inférieur et n un nombre entre 0 et 4, caractérisé en ce qu'on fait réagir les dérivés d'acide N-oxyde-2-pyridinecarboxylique correspondants de formule générale II

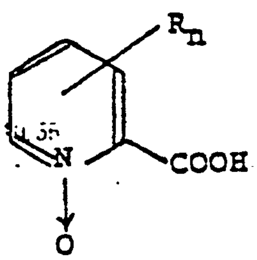

(II)

en présence d'anhydrides d'acides carboxyliques aliphatiques organiques ayant 1 à 4 atomes de C, d'alcoylamines tertiaires à base de trialcoylamines ayant 1 à 4 atomes de C dans chaque groupe alcoyle et d'un composé fournissant des halogènes de formule générale III

$CH_nX_m$ (III)

dans laquelle n=1 ou 2 et m=2 ou 3, X=halogène et n+m=4;

ou de formule générale IV

$C_2H_pX_m$ (IV)

dans laquelle p=3 ou 4 et m=2 ou 3, X=halogène et p+m=6, en excès, on règle le mélange réactionnel à une valeur de pH de 12-13 et on isole le produit désiré à partir de ce mélange.

2. Procédé selon la revendication 1, caractérisé en ce que l'on utilise, en tant qu'anhydride d'acide organique, l'anhydride acétique.

3. Procédé selon les revendications 1 et 2, caractérisé en ce que l'on utilise en tant qu'alcoylamine tertiaire, la triétylamine.

4. Procédé selon les revendications 1 - 3, caractérisé en ce que l'on utilise comme composé

fournissant des halogènes $CH_2X_2$ avec X représentant chlore, iode ou brome.

5. Procédé selon les revendications 1 - 4, caractérisé en ce que l'on utilise le composé fournissant des halogènes en un excès de 8 - 60 équivalents, pour un équivalent de N-oxyde.

6. Procédé selon les revendications 1 - 5, caractérisé en ce que l'on utilise l'anhydride d'acide en un excès de 1,2 - 3 équivalents, pour un équivalent de N-oxyde.

7. Procédé selon les revendications 1 - 6, caractérisé en ce qu'on met d'abord l'anhydride d'acide ensemble avec l'alcoylamine tertiaire et le composé fournissant des halogènes et on ajoute de façon dosée le N-oxyde.

8. Procédé selon les revendications 1 - 6, caractérisé en ce qu'on met d'abord ensemble le N-oxyde avec l'alcoylamine tertiaire et le composé fournissant des halogènes et on ajoute l'anhydride de façon dosée.

9. Procédé selon les revendications 1 - 8, caractérisé en ce que, dans le cas où R représente un radical ou des radicaux alcoyle, on isole les 2-halogèno-pyridines par extraction à partir du mélange réactionnel.

10. Procédé selon les revendications 1 - 8, caractérisé en ce que dans le cas où R représente le groupe ou des groupes COOH, on fait précipiter les acides 2-halogénopyridinecarboxyliques par réglage du mélange réactionnel à une valeur de pH de 1,5 à 2.